# EUROPEAN PATENT SPECIFICATION

(11) **EP 3 436 797 B1**
(45) Date of publication and mention of the grant of the patent: **11.11.2026**
(21) Application number: 17814362.4
(22) Date of filing: 31.03.2017
(51) Int. Cl.: G01N 1/40, B81B 3/00, B82Y 25/00, G01N 33/53

(54) **DMF METHOD AND SYSTEM FOR CONCENTRATING ANALYTE FROM LARGE VOLUMES INTO SMALLER VOLUMES USING MAGNETIC MICROPARTICLES**
DMF-VERFAHREN UND SYSTEM ZUR KONZENTRIERUNG EINES ANALYTEN AUS GROSSEN VOLUMINA IN KLEINERE VOLUMINA MIT MAGNETISCHEN MIKROPARTIKELN
PROCÉDÉ ET SYSTÈME DMF PERMETTANT DE CONCENTRER UN ANALYTE À PARTIR DE GRANDS VOLUMES EN VOLUMES PLUS PETITS À L'AIDE DE MICROPARTICULES MAGNÉTIQUES

(30) Priority: 01.04.2016 US 201662317042 P
(43) Date of publication of application: 06.02.2019
(73) Proprietor: The Governing Council of the University of Toronto, Toronto, Ontario M5G 1L7 (CA)
(72) Inventor: RACKUS, Darius George, 4051 Basel (CH); WHEELER, Aaron Ray, Toronto Ontario M6H 3M4 (CA)
(74) Representative: FRKelly
(86) International application number: PCT/CA2017/050399
(87) International publication number: WO 2017/219122

(56) References cited:
- WO-A2-2007/041692
- WO-A2-2014/113598
- US-A1- 2009 283 407
- US-A1- 2010 123 457
- US-A1- 2015 377 831
- KIHWAN CHOI ET AL: "Automated Digital Microfluidic Platform for Magnetic-Particle-Based Immunoassays with Optimization by Design of Experiments", ANALYTICAL CHEMISTRY, vol. 85, no. 20, 26 August 2013 (2013-08-26), US, pages 9638 - 9646, XP055635108, ISSN: 0003-2700, DOI: 10.1021/ac401847x
- BRENDON SEALE ET AL: "Digital Microfluidics for Immunoprecipitation", ANALYTICAL CHEMISTRY, vol. 88, no. 20, 4 October 2016 (2016-10-04), US, pages 10223 - 10230, XP055645960, ISSN: 0003-2700, DOI: 10.1021/acs.analchem.6b02915
- NINGSI MEI ET AL: "Digital Microfluidic Platform for Human Plasma Protein Depletion", ANALYTICAL CHEMISTRY, vol. 86, no. 16, 8 August 2014 (2014-08-08), US, pages 8466 - 8472, XP055646068, ISSN: 0003-2700, DOI: 10.1021/ac5022198
- DARIUS G. RACKUS ET AL: "A digital microfluidic device with integrated nanostructured microelectrodes for electrochemical immunoassays", LAB ON A CHIP, vol. 15, no. 18, 1 January 2015 (2015-01-01), pages 3776 - 3784, XP055646004, ISSN: 1473-0197, DOI: 10.1039/C5LC00660K
- MOHTASHIM H. SHAMSI ET AL: "A digital microfluidic electrochemical immunoassay", LAB ON A CHIP, vol. 14, no. 3, 1 January 2014 (2014-01-01), pages 547 - 554, XP055645983, ISSN: 1473-0197, DOI: 10.1039/C3LC51063H
- DARIUS G. RACKUS ET AL: "Electrochemistry, biosensors and microfluidics: a convergence of fields", CHEMICAL SOCIETY REVIEWS, vol. 44, no. 15, 1 January 2015 (2015-01-01), UK, pages 5320 - 5340, XP055646010, ISSN: 0306-0012, DOI: 10.1039/C4CS00369A
- ALPHONSUS H. C. NG ET AL: "Digital Microfluidic Magnetic Separation for Particle-Based Immunoassays", ANALYTICAL CHEMISTRY, vol. 84, no. 20, 26 September 2012 (2012-09-26), US, pages 8805 - 8812, XP055645974, ISSN: 0003-2700, DOI: 10.1021/ac3020627

## Description

### FIELD

The present disclosure relates to a digital microfluidics (DMF) based method and system for concentrating analyte from large volumes into smaller volumes using magnetic- micro and nanoparticles having analyte specific receptors bound thereto.

### BACKGROUND

Magnetic micro- and nanoparticles are particles with diameters on the micro- or nanometer length-scale (hereafter "microparticles") that have magnetic or paramagnetic cores making them susceptible to manipulation by magnetic fields. The surfaces of these particles can be functionalized with specific binding elements (e.g. nucleic acids, antigens, antibodies), also referred to as analyte specific receptors. Functionalized microparticles are used in a variety of applications including immunoassays, sample cleanup, and nucleic acid assays. Temporarily immobilizing magnetic particles in a magnetic field enables a user to change the solution the particles are suspended in as well as the volume in which the particles are suspended. Concentration of magnetic microparticles is particularly useful in contexts in which (a) the target analyte to be captured on the microparticles is a solute present at low concentration or a suspended particle present at low density (e.g., circulating tumour cells), and/or (b) the method of detection does not have sufficient sensitivity to detect the captured target.¹

DMF is an emerging technology in which discrete liquid droplets are manipulated on the surface of an array of electrodes. DMF has numerous complementary differences relative to conventional enclosed-microchannel-based fluidics, including reconfigurability (a generic device format can be used for any application) and absolute control over all reagents. DMF is typically implemented in a "two-plate" format, in which droplets are sandwiched between a bottom plate (bearing an array of electrodes coated with an insulator), and a top plate (bearing a ground electrode not coated with an insulator).²

Recently, DMF has proven to be a useful tool for handling small volumes of magnetic microparticles.³⁻⁸ Its open platform eliminates the potential for particles to clog the device (unlike in microchannels) and its reconfigurability means that a single chip can be used for a variety of applications in combination with appropriately functionalized particles.

To date, one limitation of DMF chips is the inability to work with large volumes, which limits the capacity to concentrate dilute solutes or suspended particles. The area of the underlying electrodes determines fluid volume on DMF chips and the total volume capacity of the chip is the sum of the area of all the electrodes multiplied by the gap distance between the bottom and top plates. As a result, the theoretical concentration factor possible on any chip is limited both by the total area of the DMF device and the area of the smallest electrode.

In practice, the concentration factor will be smaller than this theoretical factor because of limitations around the geometry of the device and the positioning of the magnet. To effectively concentrate magnetic microparticles on a DMF device by a factor of several orders of magnitude, the device must be able to process volumes far greater than the capacity of the device.

### SUMMARY

Disclosed herein is a method for sequestering and concentrating an analyte from a volume of liquid sample to a droplet of reagent with a smaller volume. The method includes exposing magnetic microparticles coated with analyte specific receptors to the volume of liquid sample (which may be arbitrarily large) containing the analytes and incubating such that analytes are bound to receptors on the particles. The volume of liquid containing the magnetic microparticles is placed on or adjacent to electrodes on a digital microfluidic device. A virtual fluid flow channels is produced across the digital microfluidic device by activing a preselected pattern of driving electrodes with a preselected pattern of voltages across the digital microfluidic device from the reservoir to an exit location from which liquid is to be removed. At the same time a magnetic field is applied at a preselected holding location along the virtual fluid flow channel so that upon activating the preselected pattern of driving electrodes, liquid from the volume of liquid in the reservoir is moved from the reservoir along the virtual flow path. The magnetic microparticles with the analyte bound to the analyte specific receptors moving with the liquid from the reservoir, upon reaching the holding location, are held at the holding location by the magnetic field, and the remaining liquid flows to the exit location by means of a pump mechanism.

Once the magnetic particles have been pinned or held at the holding location, a droplet of a selected reagent is dispensed over the over the magnetic microparticles held at the holding location. This droplet of reagent has a much smaller volume compared to the original volume of liquid from which the analytes were extracted. The magnetic field may be removed either before the reagent droplet is added, or during addition of the droplet, or after addition of the reagent droplet. The droplet of reagent containing the magnetic microparticles is further processed by the DMF to ensure the magnetic particles are homogenously mixed with the droplet of reagent, for example by activating various driving electrodes to move the droplet around to induce mixing. The droplet of reagent containing the magnetic microparticles having the analytes bound thereto contains a higher concentration of the analyte as compared to a concentration of the analyte in the volume of liquid.

In an embodiment, the pumping mechanism may be an absorbent wicking medium (such as a tissue or piece of filter paper) located at the exit location, and once the virtual channel(s) are created by applying voltages to a series of electrodes that connect the reservoir to the exit location, the absorbent wicking medium then acts as a pump by wicking fluid through capillary forces.

A further understanding of the functional and advantageous aspects of the present disclosure can be realized by reference to the following detailed description and drawings.

### BRIEF DESCRIPTION OF THE DRAWINGS

Embodiments will now be described, by way of example only, with reference to the drawings, in which:
**Figure 1** Illustrates the capture of target analyte from a volume of liquid sample by magnetic microporaticles coated with analyte specific receptors.
**Figures 2i, 2ii, 2iii, 2iv and 2v** show a cross sectional side view illustration (not to scale) of a DMF device demonstrating steps of the present method.
**Figures 3i, 3ii, 3iii, 3iv** and **3v** show a top down view illustration of the DMF device demonstrating the five steps of the present method.
**Figure 4** shows the results of tests exploring the recovery rate of 2.8, 5, and 10 µm magnetic particles from 50, 75, and 100 µL volumes of phosphate buffered saline which presents the mean percentage recovery and the standard deviations across three tests for each condition.
**Figure 5** is a graph showing calibration curves for the standard DMF-ELISA method and the pre-concentration DMF-ELISA process disclosed herein.
**Figure** 6 shows a DMF device with hydrophilic stripes leading to and from the magnetic microparticle sequestering location.

### DETAILED DESCRIPTION

Various embodiments and aspects of the disclosure will be described with reference to details discussed below. The following description and drawings are illustrative of the disclosure and are not to be construed as limiting the disclosure. The Figures are not to scale. Numerous specific details are described to provide a thorough understanding of various embodiments of the present disclosure. However, in certain instances, well-known or conventional details are not described in order to provide a concise discussion of embodiments of the present disclosure.

As used herein, the terms, "comprises" and "comprising" are to be construed as being inclusive and open ended, and not exclusive. Specifically, when used in the specification and claims, the terms, "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately" are meant to cover variations that may exist in the upper and lower limits of the ranges of values, such as variations in properties, parameters, and dimensions. **In** one non-limiting example, the terms "about" and "approximately" mean plus or minus 10 percent or less.

Unless defined otherwise, all technical and scientific terms used herein are intended to have the same meaning as commonly understood to one of ordinary skill in the art.

As used herein, the phrase "magnetic- micro and nanoparticles" refers to particles comprising paramagnetic iron oxide cores encapsulated with a polymer shell, such as polystyrene, ranging in diameter from 1 to 10 microns and functionalized with a capture moiety. This definition also describes magnetic nanoparticles with diameters on the order of 10-50 nanometers and are functionalized with a capture moiety.

Components of the method illustrated in are identified as follows:
**Captions and labels**
   1. volume of liquid sample (containing magnetic particles with bound analyte)
   2. droplet of reagent of smaller volume
   3. magnetic microparticles coated with analyte specific receptor (and bound analyte)
   4. Digital Microfluidic Device
   5. Virtual fluid flow channel
   6. Driving electrodes
   7. Exit location
   8. Magnet/magnetic field
   9. Holding location
   10. Pump mechanism
(Pertaining to the DMF device)
   11. top plate substrate (glass)
   12. top plate electrode (indium tin oxide)
   13. hydrophobic coating (Teflon, FluoroPel)
   14. insulating dielectric (Parylene C)
   15. reservoir (electrode)
   16. bottom plate substrate (glass)
(Miscellaneous)
   17. magnetic lens
   18. "top plate"
   19. volume of reagent
   20. analyte specific receptor
   21. analyte
   22. hydrophilic stripe extending from the reservoir
   23. hydrophilic stripe extending from the exit

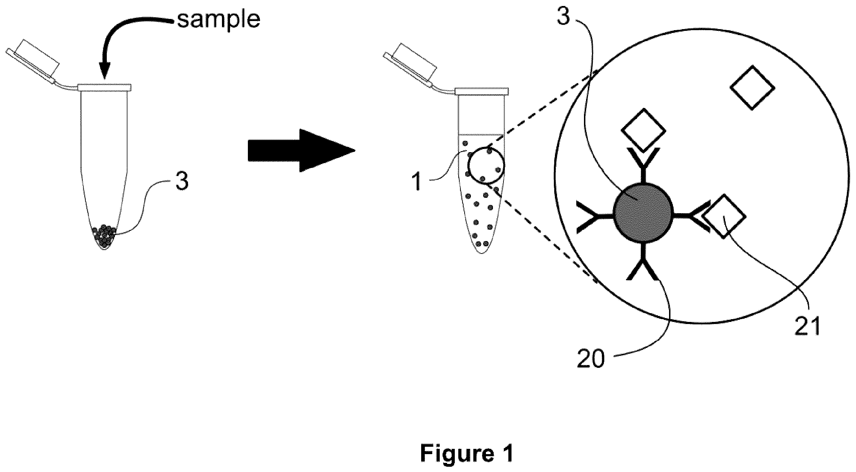

**Figure 1** Illustrates the capture of target analyte **21** from a volume of liquid sample **1** by magnetic microparticles **3** coated with analyte specific receptors **20.** As can be seen, the method involves providing the magnetic microparticles 3 in a container and adding the large volume of liquid sample 1 into the container whereupon any analytes **21** present in the sample bind to their complimentary analyte specific receptor **20** bound to the magnetic particle.

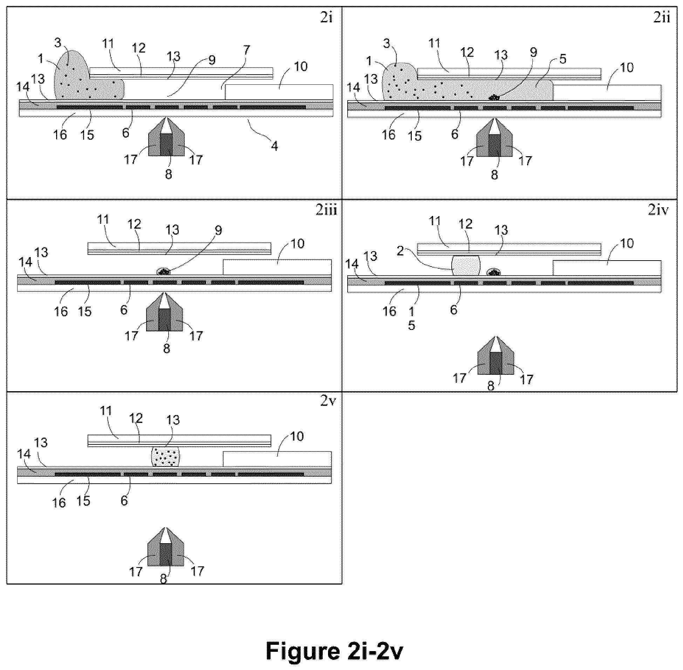

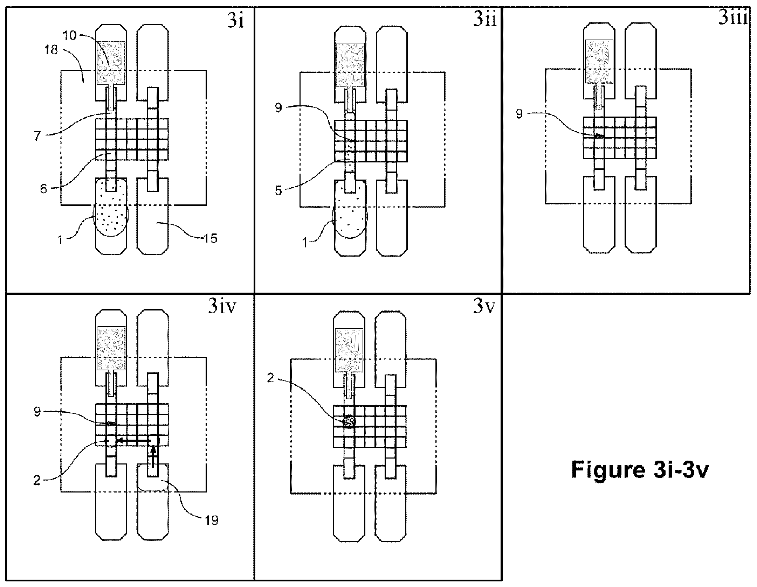

As can be seen from **Figures 2i to 2v** inclusive, DMF device **4** includes a pattern of driving electrodes **6,** which when activated in a preselected pattern may be used to define a virtual fluid flow channel **5 (****Figure 3ii****)** across the DMF device **4** from reservoir **15** (which has its own set of electrodes) to an exit location **7.** A magnet **8** with a lensing or field guiding structure **17** is located under preselected driving electrode(s) **6** to generate magnetic field above the preselected driving electrodes **6.** The preselected location of the magnetic field defines a holding location **9** along the virtual fluid flow channel **5** above the preselected driving electrode(s) **6** such that upon engagement of the magnetic field any magnetic microparticles **3** passing along the flow channel **5** are immobilized by the magnetic field while the liquid of the sample continues to flow.

DMF device **4** includes a pump mechanism **10** located in one or more preselected position(s) **7** away from the electrodes **6** such that the liquid flowing along the virtual flow channel **5** is drawn off the DMF device **4** into a waste container. The pump mechanism **10** can be an active pump such as, but not limited to, a syringe, a peristaltic pump, or vacuum pump or a passive pump such as, but not limited to, an absorbent wicking material (filter paper or tissue paper, as two non-limiting examples).

The DMF device **4** comprises a bottom plate **16** and a top plate **18** where the bottom plate **16** contains a pattern of the driving electrodes **6** and the electrodes of reservoir **15** coated with an insulating dielectric layer **14** that is covered by a layer of hydrophobic material **13** and where the top plate **18** comprises a substrate **11** with a counter electrode **12** that is covered in a layer of hydrophobic material **13.** Other potential embodiments (not shown) include reversing the orientation of plates (with "top" plate on bottom and vice versa), and/or with both plates covered with an insulating dielectric layer, and/or with multiple patterned driving and counter-electrodes on both plates, and/or in "single-plate" mode in which all driving and counter-electrodes are on a single bottom plate.

**Figures 2i, 2ii, 2iii, 2iv and 2v** show a cross sectional side view illustration (not to scale) of the DMF device **4** demonstrating the five steps of the method. In **Figure 2i** the volume of liquid sample **1** containing the magnetic micropartilces **3** which have analyte **21 (****Figure 1****)** bound thereto is placed on the DMF device **4** at a loading reservoir **15.**

In **Figure 2ii** a virtual fluid flow channel **5** is formed by actuating a sequence of driving electrodes **6** connecting the volume of liquid sample **1** with the pump mechanism **10** located at an exit location **7** to remove the liquid from the DMF device **4.** Simultaneously the magnet **8** which has been prepositioned to apply a magnetic field to the holding location **9** under the preselected driving electrodes 6 and is engaged thereby immobilizing the magnetic particles **3** coated with analyte specific receptors **20** and bound analyte **21** in the holding location **9.**

**In** **Figure 2iii** the volume of liquid **1** has been removed by the pump mechanism **10** leaving behind the magnetic particles **3** coated with analyte specific receptors **20** and bound analyte **21** in the holding zone **9.**

**In** **Figure 2iv** the magnet **8** is moved away from the DMF device **4** thereby removing the magnetic field and a droplet of reagent **2** of smaller volume is dispensed onto the magnetic microparticles **3** in the holding location **9.**

**In** **Figure 2v****,** the droplet of reagent **2** of smaller volume mixes with the magnetic microparticles **3** coated with analyte specific receptors **20** and bound analyte **21.**

**Figures 3i, 3ii, 3iii, 3iv** and **3v** show a top down view illustration of the DMF device **4** demonstrating the five steps of the method.

In **Figure 3i** the volume of liquid sample **1** is placed on one or more of the reservoirs **15** outside of the top plate **18** and a portion of the volume of liquid sample **1** is drawn underneath the top plate **18.** The pump mechanism **10** is situated underneath the top plate **18** and is adjacent to the exit location **7.**

In **Figure 3ii** the virtual fluid flow channel **5** is formed by activating the driving electrodes **6** connecting the volume of liquid sample **1** with the pump mechanism **10** located at an exit location **7** to remove the liquid from the DMF device **4.** Simultaneously the magnet **8** which has been prepositioned to apply a magnetic field to the holding location **9** under the preselected driving electrodes **6** and is engaged thereby immobilizing the magnetic particles **3** coated with analyte specific receptors **20** and bound analyte **21** in the holding location **9.**

In **Figure 3iii** the pump mechanism **10** has removed the volume of liquid sample **1** leaving the magnetic microparticles **3** coated with analyte specific receptor **20** and bound analyte **21** in the holding location **9.**

In **Figure 3iv** reagent **19** (the same as reagent **2** but a larger volume in the reservoir 15) is loaded into one of the reservoirs **15** and a smaller droplet **2** of reagent is dispensed by actuating a series of preselected driving electrodes **6.**

In **Figure 3v** the smaller droplet **2** of reagent is mixed with the magnetic microparticles **3** coated with analyte specific receptors **20** and bound analyte **21** by actuating preselected driving electrodes **6** thereby causing mixing.

### Results

In preliminary tests, 100 µL solutions containing magnetic microparticles at a density of 1.04 ×10⁷ particles per mL were processed using the described method. The immobilized magnetic particles were resuspended in approximately 1.8 µL of buffer solution. The resulting density was measured to be 4.75 ± 0.37 ×10⁸ particles per mL, a concentration factor of approximately 45-fold. In theory, concentration factors of 100-fold and greater should be attainable by this described method, dependent only on the volume of liquid that can be added to the reservoir.

The absorbent wicking material can be chosen in order to control the flow rate of the virtual channel. Both the material and the geometry of the wick affect flow rates. In tests where 75 µL solutions were processed on a DMF device using a virtual channel and an absorbent wick, materials such as a double stack of 10 mm × 10 mm Whatman No. 1 filter paper imbibed 75 µL in 60 seconds whereas a more absorbment material such as double stack of 10 mm × 10 mm SureWick G028 glass fiber imbibes the same volume in 7 seconds.

In an alternative embodiment of the present method a one-plate DMF device where the driving electrodes and counter electrode are coplanar may be used. The one-plate device differs from the two-plate device in how the voltages are applied. Instead of applying the driving voltage to the bottom plate and the ground voltage to the top plate, the driving and ground voltages are both applied to adjacent electrodes on the bottom plate. In this embodiment the pre-concentration procedure remains the same as what was described above.

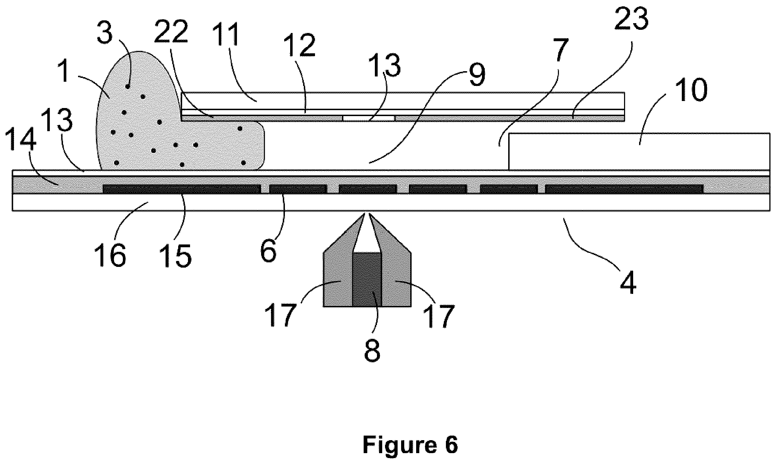

Another embodiment of the present method relies on using a two plate DMF device with hydrophilic patterns on the top plate. This is illustrated in **Figure 6****.** In this embodiment one hydrophilic stripe **22** extends from the reservoir toward a pre-specified location where magnetic microparticles are to be sequestered on the DMF device. A second hydrophilic stripe **23** extends from the exit toward the same pre-specified location such that there is a gap between the two hydrophilic stripes which is less than the length of the underlying driving electrode on the bottom plate at the location where sequestering takes place. After loading the liquid volume sample, it is wicked along to the pre-specified location by the first hydrophilic stripe. By applying a voltage to the driving electrode at the pre-specified location, the liquid sample is bridged to the second hydrophilic stripe and imbibed by the passive pump.

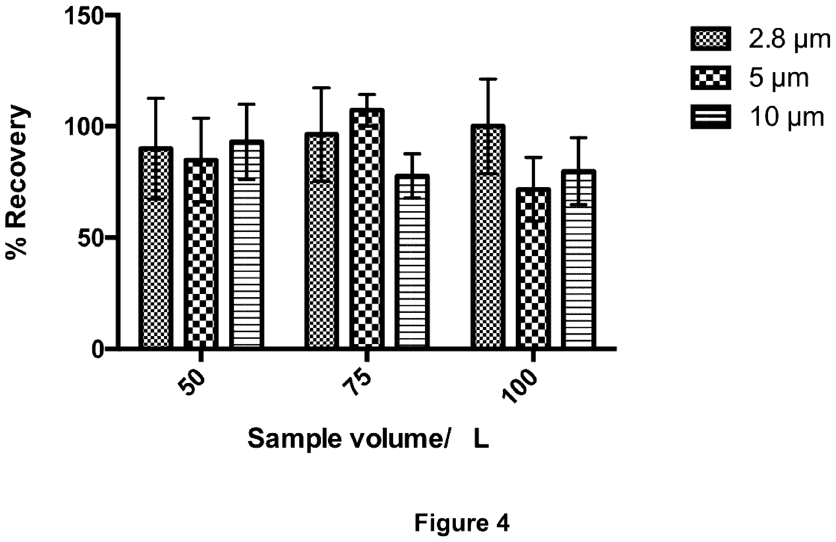

The method of pre-concentrating magnetic particles on DMF was applied to different sample liquids, including phosphate buffered saline, saliva, and urine. In all cases, the method was capable of removing the supernatant liquid and concentrating the particles. The results of further tests exploring the recovery rate of 2.8, 5, and 10 µm magnetic particles from 50, 75, and 100 µL volumes of phosphate buffered saline are shown in **Figure 4****.** In this test, 2 µL volumes of magnetic particles at densities of 9.00 × 10⁷ to 1.84 × 10⁸ were added to a volume of phosphate buffered saline. Particles were then concentrated using the pre-concentration method described here, and recovered particles were counted. **Figure 4** presents the mean percentage recovery and the standard deviations across three tests for each condition.

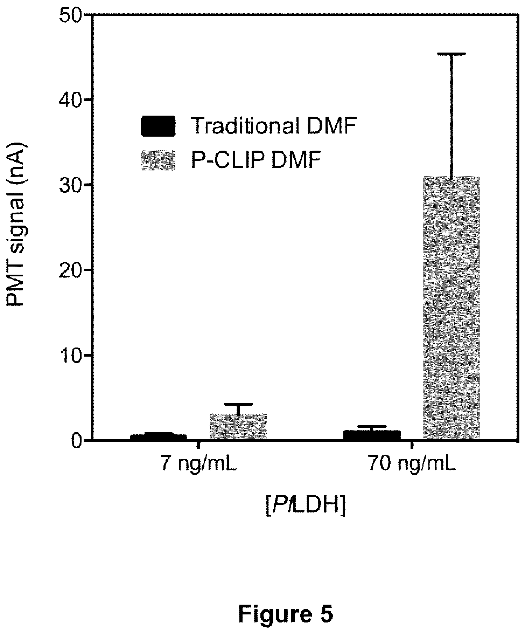

The pre-concentration of particles can be used to improve the sensitivities of capture assays such as immunoassays or nucleic acid hybridization assays. A DMF enzyme-linked immunosorbent assay (ELISA) for *Plasmodium falciparum* lactate dehydrogenase (LDH) was performed on-chip using conventional DMF-ELISA protocols and a protocol modified by the addition of the pre-concentration method. A conventional DMF-ELISA was run as a comparison where a 2.4 µL volume of magnetic particles (6.7 × 10⁸ particles/mL) functionalized with anti-*Pf*LDH antibodies was dispensed on the DMF device. The particles were immobilized and the supernatant was removed. The particles were then incubated with 2.4 µL of phosphate buffered saline and the antigen *Pf*LDH. The mixture was incubated for 5 minutes with mixing by the DMF electrodes. The particles were then subjected to the standard DMF-ELISA protocol of washing, incubating with enzyme-conjugated antibody labels, further washing, incubating with chemiluminescent enzyme substrates (luminol and H₂O₂), and measuring chemiluminescence with a photomultiplier tube. For the pre-concentration method, a 2.4 µL volume of magnetic particles functionalized with anti-*Pf*LDH antibodies was added to a microcentrifuge tube containing 75 µL phosphate buffered saline and the antigen *Pf*LDH. The mixture was incubated for 3 hours with rotation at room temperature before being processed on a DMF device using the pre-concentration method described here. A longer incubation time was necessary for the pre-concentration method due to the 30-fold decrease in particle concentration. The particles were then subjected to the standard DMF-ELISA protocol. Results comparing the signals from pre-concentration to the conventional DMF-ELISA for concentrations of 7 ng/mL and 70 ng/mL *Pf*LDH are presented in **Figure 5****.** Signal increases of up to 30-fold were observed.

### Discussion

Approaches to magnetic particle-based capture bioassays by DMF involve mixing magnetic microparticles coated with capture agents and a sample on the DMF device. This conventional approach involves dispensing a volume of magnetic particles, removing the supernatant liquid, dispensing a similar-sized volume of sample and mixing the droplet of sample with the magnetic microparticles. The amount of target analyte that can be captured is limited to what is present in the volume of the sample and these volumes are typically on the order of 50 nL to 5 µL. This may be sufficient for detecting a certain range of concentrations of the target analyte within the sample, but will not be sufficient for detecting concentrations of analyte which are below the limit of detection of the analytical detector.

To overcome the challenge of insufficient analyte within the sample, larger volumes can be used, thereby capturing more analyte. This proves difficult to process using the conventional DMF method described above as the maximum volume that can be processed at a time is limited by the size of the DMF device. This could be circumvented by repeatedly mixing the magnetic particles with a droplet of sample, removing the sample from the particles by means of a magnet and repeating the incubation with another droplet. This repetitive process is time consuming, inefficient and requires multiple steps.

Another approach to processing large volumes of sample on a DMF device would be to create a virtual channel defined by a series of driving electrodes and powered by a pump and to use this channel to flow a larger volume of sample over magnetic particles that have been immobilized by a magnetic field on the device. While this method allows larger volumes of sample to be processed, the immobilized particles are clustered within the magnetic field and a limited surface of the particles is exposed to the sample thereby reducing the amount of analyte that is bound. This is inefficient as many of the available binding sites on the magnetic particle are buried within the clump and not exposed to the sample.

The present method disclosed herein overcomes the problem of processing large volumes of sample with magnetic microparticles for bioassays by performing the incubation of the magnetic microparticles with the sample off-chip and then using a combination of a virtual channel on the DMF device and a magnet to concentrate the analytes captured from the larger liquid sample. The volume of sample that can be processed by this method is on the order of 50 to 1000 times greater than the volumes typically processed by DMF and concentrates the analytes into a smaller volume that is amenable to further processing by a DMF device.

Further, when the pump mechanism that drives the virtual channel on the device is a passive pump, such as an absorbent wicking material like filter paper or tissue paper, the need for complicated active pumps and tubing is eliminated. This simplification even allows for the passive pump to be preloaded on the DMF device.

While the present method as exemplified above has been described with respect to increasing the concentration of an analyte of interest which may be present in low concentrations difficult to detect, it will be appreciated that the present method may also be used to screen out or remove analytes which may be considered interferents which when present in high concentrations mask the presence of analytes which need to be detected. In this embodiment the volume of liquid sample that is removed from the magnetic microparticles coated with analyte specific receptors and bound analyte is retained in a container and can be reintroduced to the DMF device for further processing and analysis or delivered to another system for analysis.

### References

1. K. Aguilar-Arteaga, J. A. Rodriguez and E. Barrado, Analytica Chimica Acta, 2010, 674, 157-165.
2. K. Choi, A. H. C. Ng, R. Fobel and A. R. Wheeler, in Annual Review of Analytical Chemistry, Vol. 5, eds. R. G. Cooks and E. S. Yeung, Annual Reviews, Palo Alto, 2012, vol. 5, pp. 413-440.
3. K. Choi, A. H. C. Ng, R. Fobel, D. A. Chang-Yen, L. E. Yarnell, E. L. Pearson, C. M. Oleksak, A. T. Fischer, R. P. Luoma, J. M. Robinson, J. Audet and A. R. Wheeler, Analytical Chemistry, 2013, 85, 9638-9646.
4. N. M. Lafreniere, J. M. Mudrik, A. H. C. Ng, B. Seale, N. Spooner and A. R. Wheeler, Analytical Chemistry, 2015, 87, 3902-3910.
5. N. S. Mei, B. Seale, A. H. C. Ng, A. R. Wheeler and R. Oleschuk, Analytical Chemistry, 2014, 86, 8466-8472.
6. A. H. C. Ng, K. Choi, R. P. Luoma, J. M. Robinson and A. R. Wheeler, Analytical Chemistry, 2012, 84, 8805-8812.
7. A. H. C. Ng, M. Lee, K. Choi, A. T. Fischer, J. M. Robinson and A. R. Wheeler, Clinical Chemistry, 2015, 61, 420-429.
8. M. H. Shamsi, K. Choi, A. H. C. Ng and A. R. Wheeler, Lab on a Chip, 2014, 14, 547-554.

## Claims

1. A method for sequestering and concentrating an analyte from a volume of liquid sample to a droplet of reagent with a smaller volume, comprising:
(a) exposing magnetic microparticles coated with analyte specific receptors to the volume of liquid sample containing the analytes;
(b) placing the volume of liquid containing the magnetic microparticles into a reservoir forming part of a digital microfluidic device;
(c) forming a virtual fluid flow channel across the digital microfluidic device by activating a preselected pattern of driving electrodes with a preselected pattern of voltages across the digital microfluidic device from the reservoir to an exit location from which liquid is to be removed from the digital microfluidic device and at the same time applying a magnetic field at a preselected holding location along the virtual fluid flow channel, wherein upon activating the preselected pattern of driving electrodes, liquid from the volume of liquid in the reservoir traverses a distance from the reservoir to the exit location and the magnetic microparticles with the analyte bound to the analyte specific receptors moving from the reservoir, upon reaching the holding location, are substantially held at the holding location by the magnetic field, and the remaining liquid remains flowing to the exit location by pumping to be removed from the digital microfluidic device;
(d) dispensing a droplet of a reagent over the magnetic microparticles held at the holding location by the magnetic field, said droplet of reagent having a smaller volume compared to the volume of liquid; and
(e) removing the magnetic field at the holding location either before, during or after step d) and further processing the droplet of reagent containing the magnetic microparticles; wherein the dispensing step (d) produces the droplet of reagent containing the magnetic microparticles having the analytes bound thereto that contains a higher concentration of analyte as compared to a concentration of the analyte in said volume of liquid sample.

2. The method according to claim 1 wherein the pumping is carried out by passive pumping.

3. The method according to claim 2 wherein the passive pumping is by means of an absorbent wicking medium.

4. The method according to claim 1 wherein the pumping is carried out by active pumping.

5. The method according to any one of claims 1 to 4 wherein the method uses a one plate digital microfluidic device.

6. The method according to any one of claims 1 to 4 wherein the method uses a two plate digital microfluidic device.

7. The method according to any one of claims 1 to 6 wherein the method uses the digital microfluidic device that includes one hydrophilic stripe extending from the reservoir toward a pre-specified location where magnetic microparticles are to be sequestered on the DMF device, and including a second hydrophilic stripe extending from the exit toward the same pre-specified location such that there is a gap between the two hydrophilic stripes which is less than a length of the underlying driving electrode on the bottom plate at the location where sequestering takes place, and wherein after loading the liquid volume sample, it is wicked along to the pre-specified location by the first hydrophilic stripe, and by applying a voltage to the driving electrode at the pre-specified location, the liquid sample is bridged to the second hydrophilic stripe and imbibed by the pumping mechanism.

## Patentansprüche

1. Verfahren zum Sequestrieren und Konzentrieren eines Analyten aus einem Volumen flüssiger Probe zu einem Tröpfchen von Reagenz mit einem kleineren Volumen, umfassend:
(a) Aussetzen magnetischer Mikropartikel, die mit analytenspezifischen Rezeptoren beschichtet sind, gegenüber dem Volumen flüssiger Probe, das die Analyten enthält;
(b) Einbringen des Volumens von Flüssigkeit, das die magnetischen Mikropartikel enthält, in ein Reservoir, das einen Teil einer digitalen mikrofluidischen Vorrichtung bildet;
(c) Bilden eines virtuellen Fluidströmungskanals über die digitale mikrofluidische Vorrichtung durch ein Aktivieren eines vorausgewählten Musters von Ansteuerelektroden mit einem vorausgewählten Muster von Spannungen über die digitale mikrofluidische Vorrichtung von dem Reservoir zu einer Austrittsstelle, von der Flüssigkeit aus der digitalen mikrofluidischen Vorrichtung zu entfernen ist, und gleichzeitig ein Anlegen eines Magnetfelds an einer vorausgewählten Haltestelle entlang des virtuellen Fluidströmungskanals, wobei nach Aktivieren des vorausgewählten Musters von Ansteuerelektroden Flüssigkeit aus dem Volumen von Flüssigkeit in dem Reservoir eine Distanz von dem Reservoir zu der Austrittsstelle zurücklegt und die magnetischen Mikropartikel, die sich aus dem Reservoir bewegen, mit dem Analyten, der an die analytenspezifischen Rezeptoren gebunden ist, nach einem Erreichen der Haltestelle durch das Magnetfeld im Wesentlichen an der Haltestelle gehalten werden, und die restliche Flüssigkeit durch ein Pumpen weiter zu der Austrittsstelle fließt, um aus der digitalen mikrofluidischen Vorrichtung entfernt zu werden;
(d) Abgeben eines Tröpfchens eines Reagenzes über die magnetischen Mikropartikel, die durch das Magnetfeld an der Haltestelle gehalten werden, wobei das Tröpfchen von Reagenz im Vergleich zu dem Volumen von Flüssigkeit ein kleineres Volumen aufweist; und
(e) Entfernen des Magnetfelds an der Haltestelle entweder vor, während oder nach Schritt d) und ein Weiterverarbeiten des Tröpfchens von Reagenz, das die magnetischen Mikropartikel enthält; wobei der Abgabeschritt (d) das Tröpfchen von Reagenz produziert, das die magnetischen Mikropartikel enthält, an die die Analyten gebunden sind, das eine höhere Konzentration von Analyt im Vergleich zu einer Konzentration des Analyten in dem Volumen flüssiger Probe enthält.

2. Verfahren nach Anspruch 1, wobei das Pumpen durch ein passives Pumpen ausgeführt wird.

3. Verfahren nach Anspruch 2, wobei das passive Pumpen mittels eines absorbierenden Dochtwirkungsmediums erfolgt.

4. Verfahren nach Anspruch 1, wobei das Pumpen durch ein aktives Pumpen ausgeführt wird.

5. Verfahren nach einem der Ansprüche 1 bis 4, wobei das Verfahren eine einplattenbasierte digitale mikrofluidische Vorrichtung verwendet.

6. Verfahren nach einem der Ansprüche 1 bis 4, wobei das Verfahren eine zweiplattenbasierte digitale mikrofluidische Vorrichtung verwendet.

7. Verfahren nach einem der Ansprüche 1 bis 6, wobei das Verfahren die digitale mikrofluidische Vorrichtung verwendet, die einen hydrophilen Streifen umfasst, der sich von dem Reservoir zu einer vorbestimmten Stelle erstreckt, an der magnetische Mikropartikel auf der DMF-Vorrichtung zu sequestrieren sind, und einen zweiten hydrophilen Streifen umfassend, der sich von dem Austritt zu derselben vorbestimmten Stelle erstreckt, so dass ein Spalt zwischen den zwei hydrophilen Streifen besteht, der kleiner als eine Länge der darunterliegenden Ansteuerelektrode auf der Bodenplatte an der Stelle ist, an der ein Sequestrieren stattfindet, und wobei nach Laden der Flüssigvolumenprobe diese durch den ersten hydrophilen Streifen entlang zu der vorbestimmten Stelle gesaugt wird, und durch ein Anlegen einer Spannung an die Ansteuerelektrode an der vorbestimmten Stelle die flüssige Probe zu dem zweiten hydrophilen Streifen überbrückt und durch den Pumpmechanismus aufgesaugt wird.

## Revendications

1. Procédé de séquestration et de concentration d'un analyte à partir d'un volume d'échantillon liquide dans une gouttelette de réactif de volume inférieur, comprenant :
(a) l'exposition des microparticules magnétiques recouvertes de récepteurs spécifiques à l'analyte au volume d'échantillon liquide contenant les analytes ;
(b) le placement du volume de liquide contenant les microparticules magnétiques dans un réservoir faisant partie d'un dispositif microfluidique numérique ;
(c) la formation d'un canal virtuel d'écoulement de fluide à travers le dispositif microfluidique numérique en activant un modèle présélectionné d'électrodes de commande avec un modèle présélectionné de tensions à travers le dispositif microfluidique numérique, depuis le réservoir jusqu'à un emplacement de sortie d'où le liquide doit être retiré du dispositif microfluidique numérique, et en appliquant simultanément un champ magnétique à un emplacement de maintien présélectionné le long du canal virtuel d'écoulement de fluide, dans lequel, lors de l'activation du modèle présélectionné d'électrodes de commande, le liquide du volume de liquide dans le réservoir parcourt une distance depuis le réservoir jusqu'à l'emplacement de sortie, et, lorsqu'elles atteignent l'emplacement de maintien, les microparticules magnétiques avec l'analyte lié aux récepteurs spécifiques à l'analyte qui se déplacent depuis le réservoir sont sensiblement maintenues à l'emplacement de maintien par le champ magnétique, et le liquide restant continue de s'écouler vers l'emplacement de sortie par pompage pour être retiré du dispositif microfluidique numérique ;
(d) la distribution d'une goutte de réactif sur les microparticules magnétiques maintenues à l'emplacement de maintien par le champ magnétique, ladite goutte de réactif présentant un volume inférieur au volume du liquide ; et
(e) la suppression du champ magnétique à l'emplacement de maintien avant, pendant ou après l'étape (d) et le traitement ultérieur de la gouttelette de réactif contenant les microparticules magnétiques ; dans lequel l'étape de distribution (d) produit la gouttelette de réactif contenant les microparticules magnétiques auxquelles sont liés les analytes qui contient une concentration d'analyte plus élevée que la concentration de l'analyte dans ledit volume d'échantillon liquide.

2. Procédé selon la revendication 1, dans lequel le pompage est effectué par pompage passif.

3. Procédé selon la revendication 2, dans lequel le pompage passif est effectué au moyen d'un milieu absorbant par capillarité.

4. Procédé selon la revendication 1, dans lequel le pompage est effectué par pompage actif.

5. Procédé selon l'une quelconque des revendications 1 à 4, dans lequel le procédé utilise un dispositif microfluidique numérique à une seule plaque.

6. Procédé selon l'une quelconque des revendications 1 à 4, dans lequel le procédé utilise un dispositif microfluidique numérique à deux plaques.

7. Procédé selon l'une quelconque des revendications 1 à 6, dans lequel le procédé utilise le dispositif microfluidique numérique comportant une bande hydrophile s'étendant du réservoir vers un emplacement prédéfini où des microparticules magnétiques doivent être séquestrées sur le dispositif DMF, et comportant une seconde bande hydrophile s'étendant de la sortie vers le même emplacement prédéfini, de sorte qu'il existe un espace entre les deux bandes hydrophiles qui est inférieur à la longueur de l'électrode de commande sous-jacente sur la plaque inférieure à l'emplacement où la séquestration a lieu, et dans lequel, après chargement de l'échantillon de volume liquide, celui-ci est acheminé par capillarité jusqu'à l'emplacement prédéfini par la première bande hydrophile, et, en appliquant une tension à l'électrode de commande à l'emplacement prédéfini, l'échantillon liquide est relié à la seconde bande hydrophile et imbibé par le mécanisme de pompage.
